# EUROPÄISCHE PATENTSCHRIFT

(11) **EP 2 082 776 B1**
(45) Veröffentlichungstag und Bekanntmachung des Hinweises auf die Patenterteilung: **09.12.2015**
(21) Anmeldenummer: 08171042.8
(22) Anmeldetag: 09.12.2008
(51) Int. Cl.: A61F 2/958, A61M 25/10

(54) **Mehrfachmembranballon und Verfahren zur Herstellung eines Mehrfachmembranballons**
Multiple membrane balloon and method for manufacturing a multiple membrane balloon
Ballon à membranes multiples et procédé de fabrication d'un ballon à membranes multiples

(30) Priorität: 25.01.2008 DE 102008006092
(43) Veröffentlichungstag der Anmeldung: 29.07.2009
(73) Patentinhaber: Biotronik VI Patent AG, 6341 Baar (CH)
(72) Erfinder: Wesselmann, Matthias, 8912 Glattfelden (CH)
(74) Vertreter: Galander, Marcus

(56) Entgegenhaltungen:
- EP-A- 0 399 712
- EP-A- 0 903 121
- US-A- 5 087 394

## Beschreibung

Die Erfindung betrifft einen Mehrfachmembranballon und ein Verfahren zur Herstellung eines Mehrfachmembranballons nach den Oberbegriffen der unabhängigen Ansprüche.

Mehrfachmembranballons kommen bekanntermaßen bei Ballonkathetern zum Einsatz. Ein Anwendungsgebiet betrifft z.B. die Angioplastie, bei der Ballonkatheter in Blutgefäße eingeführt, bis zu einer Stenose eingeschoben und dort aufgeweitet werden, um die Verengung des Blutgefäßes zu beseitigen. Üblicherweise ragt am distalen Ende ein Führungsdraht mit kleinem Durchmesser über den Ballonkatheter hinaus. Der Ballonkatheter folgt dem Führungsdraht und schmiegt sich an den Verlauf des betreffenden Gefäßes an. Problematisch ist es, wenn der Ballon aufgrund von Beschädigungen platzt.

Bekannt ist auch, einen Mehrfachmembranballon mit einem steifen Stützorgan (Stent) zu umgeben, das als Stützgerüst im Bereich der Stenose verbleiben kann. Zum Aufweiten des Stents werden z.B. eines oder beide der Lumen der ineinander angeordneten Membranhüllen des Doppelmembranballons mit Gas oder Flüssigkeit beschickt und aufgeweitet, worauf sich der Stent auf einen größeren Durchmesser aufweitet. Ein derartiges Einführsystem für einen Stent mit einem Mehrfachmembranballon ist beispielsweise in der US 5,769,817A beschrieben.

In der WO 2007/053967 A1 ist ein Einführsystem für einen Stent beschrieben, bei dem Ballonmaterialien eingesetzt werden, welche eine günstige Gleitfähigkeit gegeneinander aufweisen. Zur Verbesserung der Gleitfähigkeit kann zwischen den Membranlagen eine gleitfähige Beschichtung vorgesehen sein. Die Gleitfähigkeit der Ballonmembranen gegeneinander erlaubt eine bessere Anpassbarkeit des Mehrfachmembranballons an einen spezifischen Verlauf eines Hohlorgans beim Einführen des Systems. Dabei können beide Lumen aufgeweitet werden, oder der Membranballon kann als doppelwandige Ausführung eingesetzt werden, bei dem nur das Lumen des inneren Ballons aufgeweitet bzw. aufgeblasen wird. Die Membranlagen liegen dann direkt aufeinander und sind gegenseitig gleitfähig. Zum Aufweiten der Mehrfachmembranballone kommen relativ hohe Drücke zum Einsatz, die bei dem beschriebenen Einführsystem zwischen 50 und 60 bar liegen.

Problematisch ist, dass der Stent, der um den Einfach -oder Mehrfachmembranballon gelegt ist, beim Befestigen desselben die äußere Membranlage leicht verletzen kann, wenn dieser nach dem Aufschieben auf den Ballon auf einen kleinen, zum Einführen des Stents geeigneten Durchmesser komprimiert wird. Wird dann der undichte Ballon am Einsatzort aufgeweitet, kann im ungünstigsten Fall der Stent nur teilweise geöffnet werden und so die Arterie blockieren.

In der EP 0 903 121 A1 ist ein Einführsystem für einen Stent mit zwei koaxial angeordneten Ballons beschrieben. Die Ballone haben separate Lumen, um sie unabhängig voneinander dilatieren zu können. Ein zu implantierender Stent ist auf dem System innerhalb einer rückziehbaren Hülle angebracht. Am Behandlungsort wird die Hülle zurückgezogen und der Stent wird mit Hilfe der Dilatationsballons platziert.

Problematisch ist, dass durch die Reibung zwischen Stent und Hülle das Zurückziehen der Hülle den Stent in seiner Lage verändern kann. Der Stent würde dann nicht mehr am vorgesehenen Ort platziert werden und die Behandlung der Stenose wäre unzureichend oder verfehlt. Darüber hinaus kann auf dem Weg zum Implantationsort die Stent mitsamt der Hülle auf dem Einführsystem verrutschen, oder aber durch z. B. Einreißen der Hülle der Stent allein verrutschen.

Der Erfindung liegt die Aufgabe zugrunde, einen Mehrfachmembranballon anzugeben, der eine höhere Sicherheit beim Einführen und Aufweiten des Ein- oder Mehrfachmembranballons gewährleistet. Weiterhin soll ein Mehrfachmembranballon angegeben werden, mit dem das Einführen eines Stents in ein Hohlorgan verbessert werden kann. Ebenso liegt der Erfindung die Aufgabe zugrunde, einen Mehrfachmembranballon anzugeben, bei dem ein darauf angebrachtes Stützorgan nicht ver- oder abrutschen kann. Ferner soll ein Verfahren zur Herstellung eines Mehrfachmembranballons angegeben werden.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Die Erfindung geht aus von einem Mehrfachmembranballon für einen Katheter mit einer wenigstens doppelten Membranhülle, umfassend wenigstens einen befüll- und entleerbaren Ausdehnungsraum zwischen einem proximalen Ende und einem distalen Ende, wobei die äußere Membranhülle eine oder mehrere innere Membranhüllen umschließt.

Es wird vorgeschlagen, dass die äußere Membranhülle an ihrem distalen Ende und ihrem proximalen Ende verschlossen ist. Eine Beschädigung der inneren Membranhülle, welche am Einsatzort aufgeweitet werden soll, kann durch die äußere Membranhülle vorteilhaft kompensiert werden. Durch die Mehrschichtigkeit kann die äußere Membranhülle, gegebenenfalls auch die innere Membranhülle, vorteilhafterweise sehr dünn, z.B. bis zu 3 µm, ausgeführt werden. Die äußere Membranhülle bildet einen abgeschlossenen Bereich, der die eine oder mehreren inneren Membranhüllen schützt. Selbst bei Vorliegen einer kleinen Undichtigkeit (Pinhole) in einer der äußeren Membranhüllen kann am Einsatzort die Dichtigkeit der inneren Membranhülle des Mehrfachmembranballons ausgenutzt werden. Bei einem Platzen der inneren Membranhülle(n) kann die freiwerdende Energie durch die äußere Membranhülle gedämpft werden und Beschädigungen des Gewebes am Einsatzort vermieden werden. Risse in einer Membranhülle können sich nicht in die andere Membranhülle fortsetzen.

Vorteilhaft kann ein die Membranhülle umgebendes Stützorgan wenigstens teilweise von der äußeren Membranhülle durchsetzt sein. Vorzugsweise kann die äußere Membranhülle formschlüssig mit dem Stützorgan verbunden sein. Dadurch kann eine Abzugskraft des Stützorgans erhöht werden, so dass ein ungewolltes Abrutschen des Stützorgans von der Membranhülle weitgehend vermieden werden kann.

Die Erfindung geht weiterhin aus von einem Mehrfachmembranballon für einen Katheter, mit einer wenigstens doppelten Membranhülle, umfassend wenigstens einen befüll- und entleerbaren Ausdehnungsraum zwischen einem proximalen Ende und einem distalen Ende, wobei ein steifes Stützorgan die wenigstens doppelte Membranhülle umschließt und die äußere Membranhülle eine oder mehrere innere Membranhüllen umschließt.

Es wird weiterhin vorgeschlagen, dass das Stützorgan wenigstens teilweise von der äußeren Membranhülle durchsetzt ist. Vorteilhaft kann eine Abzugskraft des Stützorgans erhöht werden, so dass ein ungewolltes Abrutschen des Stützorgans von der Membranhülle weitgehend vermieden werden kann. Durch die zwei oder mehr Membranhüllen kann zuverlässig verhindert werden, dass eine Beschädigung der äußeren Membranhülle die Wirkung der einen oder mehreren innere Membranhüllen beim Aufweiten am Einsatzort behindert. Umgekehrt kann eine Beschädigung der inneren Membranhülle, welche aufgeweitet werden soll, durch die äußere Membranhülle kompensiert werden. Durch die Mehrschichtigkeit kann die äußere Membranhülle, gegebenenfalls auch die innere Membranhülle, vorteilhafterweise sehr dünn, z.B. im Bereich von 3 µm ausgeführt werden.

Vorzugsweise kann die äußere Membranhülle formschlüssig mit dem Stützorgan verbunden sein. Ist das Stützorgan mit Streben und Zwischenräumen ausgebildet, etwa als Geflecht, kann die Membranhülle in Zwischenräume des Stützorgans noppenartig eingreifen. Neben der besseren Haftung des Stützorgans auf der Membranhülle kann weiterhin das umgebende Gewebe beim Einführen des Mehrfachmembranballons in ein Organ oder Blutgefäß geschützt werden, da die das Stützorgan durchsetzende Membranhülle sich leicht dem Verlauf des Organs oder Blutgefäßes anpassen kann. Ein Druckaufbau beim Aufweiten des Mehrfachmembranballons erfolgt durch die eine oder mehreren inneren, durch die äußere Membranhülle geschützten Membranhüllen.

In vorteilhafter Weiterbildung der Erfindung kann die äußere Membranhülle an ihrem distalen Ende und ihrem proximalen Ende verschlossen sein. Dadurch bildet die äußere Membranhülle einen abgeschlossenen Bereich, der die eine oder die mehreren inneren Membranhüllen schützt. Selbst bei Vorliegen einer kleinen Undichtigkeit (Pinhole) in einer der inneren Membranhüllen kann am Einsatzort die Dichtigkeit der äußeren Membranhülle des Mehrfachmembranballons ausgenutzt werden. Bei einem Platzen der inneren Membranhülle(n) kann die freiwerdende Energie durch die äußere Membranhülle gedämpft werden und Beschädigungen des Gewebes am Einsatz vermieden werden. Risse in einer Membranhülle können sich nicht in die andere Membranhülle fortsetzen.

Der distale Verschluss der äußeren Membranhülle in axialer Richtung kann vorzugsweise überlappungsfrei hinter einem distalen Verschluss der einen oder mehreren inneren Membranhüllen liegen. Die Membranhüllen bleiben gegeneinander beweglich. Dies ist vorteilhaft für die Flexibilität des distalen Bereiches des Instruments; es wird vermieden, dass die Ballonmembran im Bereich des Konus bzw. Halses durch überlappende Verschlüsse ein starres Rohr bildet. Bevorzugt entspricht der Abstand zwischen den Verschlüssen der einfachen axialen Erstreckung des Verschlusses der inneren Hülle. Durch diese Maßnahme ist der Halsbereich des Mehrfachmembranballons, in welchem sich die Verschlüsse befinden, sehr flexibel. Der Verschluss kann z.B. durch Verschweißen gebildet werden. Sein Biegemoment kann durch separates Verschließen, insbesondere Anschweißen, der äußeren Mehrfachmembran reduziert werden.

Ein Zwischenraum zwischen der äußeren Membranhülle und der nächstfolgenden inneren Membranhülle kann vorteilhafterweise einen Druck unterhalb von Atmosphärendruck aufweisen. Dies ermöglicht ein regelrechtes Einbetten und stabiles Fixieren des Stützorgans in der äußere Membranhülle.

Bevorzugt kann die äußere Membranhülle eine größere Berstgrenze aufweisen als die eine oder die mehreren inneren Membranhüllen. Bei einem Platzen einer druckbelasteten inneren Membranhülle am Einsatzort kann die äußere Membranhülle zum einen die freiwerdende Energie dämpfen, aber auch das freiwerdende Medium aufnehmen. Eine Beschädigung von umliegendem Gewebe am Einsatzort kann vermieden werden.

Günstigerweise kann die äußere Membranhülle so gefertigt sein, dass sie beim Aufweiten der einen oder der mehreren inneren Membranhüllen denselben Dehnungszustand aufweist. Die Reckverhältnisse können beim Herstellen des Mehrfachmembranballons, vorzugsweise durch Blasformen, auf einfache Weise eingestellt werden, etwa mittels Hoop-Reckung, d.h. Reckung im Umfangsbereich. Dies ermöglicht auch unter Volllast einen homogenen Spannungsverlauf im Mehrfachmembranballon in den einzelnen Membranhüllen. Volllast bedeutet eine Zunahme des Umfangs der Membranhülle um ein Mehrfaches ihres Ausgangsumfangs beim Einführen. Die Spannungsverteilung im Ballonquerschnitt kann homogenisiert werden.

Die äußere Membranhülle kann an ihrer Außenseite wenigstens bereichsweise eine Schutzschicht aufweisen, welche die Membranhülle vor Beschädigungen schützt.

Vorzugsweise kann die äußere Membranhülle ein größeres Volumen aufweisen als die eine oder die mehreren inneren Membranhüllen im regulären aufgeweiteten Zustand. Dadurch kann sichergestellt werden, dass ein Bersten der inneren Membranhülle(n) auf jeden Fall vor dem Bersten der äußeren Membranhülle erfolgt.

Es wird ferner ein Verfahren zur Herstellung eines Mehrfachmembranballons mit einer wenigstens doppelten Membranhülle vorgeschlagen, bei dem eine äußere Membranhülle der wenigstens doppelten Membranhülle aufgeweitet wird, bis die äußere Membranhülle ein die äußere Membranhülle umgebendes Stützorgan wenigstens teilweise durchsetzt.

Vorzugsweise kann die äußere Membranhülle mit dem Stützorgan formschlüssig verbunden werden. Dies kann auf einfache Weise dadurch erfolgen, dass die äußere Membranhülle aufgeweitet wird, bis sich die Membranhülle in die Zwischenräume des Stützorgans ausdehnt.

Das mit der äußeren Membranhülle verbundene Stützorgan kann auf einen gewünschten geringeren Durchmesser komprimiert werden. Dadurch ergibt sich vorteilhaft ein geringer Durchmesser der gesamten Anordnung, und ein Einführen des Mehrfachmembranballons in ein Hohlorgan wird vereinfacht. Vorzugsweise wird das Stützorgan auf den gewünschten geringeren Durchmesser gecrimpt.

In einem weiteren günstigen Verfahrensschritt kann die äußere Membranhülle an ihrem offenen Ende verschlossen werden. Dies kann vorzugsweise durch Verschweißen erfolgen.

In vorteilhafter Weiterbildung kann die Membranhülle zuerst evakuiert und dann an ihrem offenen Ende verschlossen werden. Dadurch kann das Stützelement durch die in dessen Zwischenräume eingreifenden Teile der äußeren Membranhülle besonders sicher auf dieser Membranhülle fixiert werden.

Bevorzugt können die Membranhüllen so hergestellt werden, dass sie im aufgeweiteten Zustand einen gleichartigen Dehnungszustand einnehmen. Dadurch kann im Betrieb eine Homogenisierung des Spannungsverlaufs über dem Umfang der Membranhüllen erreicht werden.

Die Erfindung ist nachfolgend beispielhaft, anhand von in Zeichnungen dargestellten Ausführungsbeispielen, näher erläutert. Es zeigen in schematischer Darstellung:
- Fig. 1: einen Schnitt durch einen bevorzugten Doppelmembranballon in einem ersten Verfahrensschritt;
- Fig. 2: einen Schnitt durch den bevorzugte Doppelmembranballon aus Fig. 1 in einem zweiten Verfahrensschritt; und
- Fig. 3: einen Schnitt durch einen weiteren bevorzugten Doppelmembranballon gemäß einem weiteren, unabhängigen Aspekt der Erfindung.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

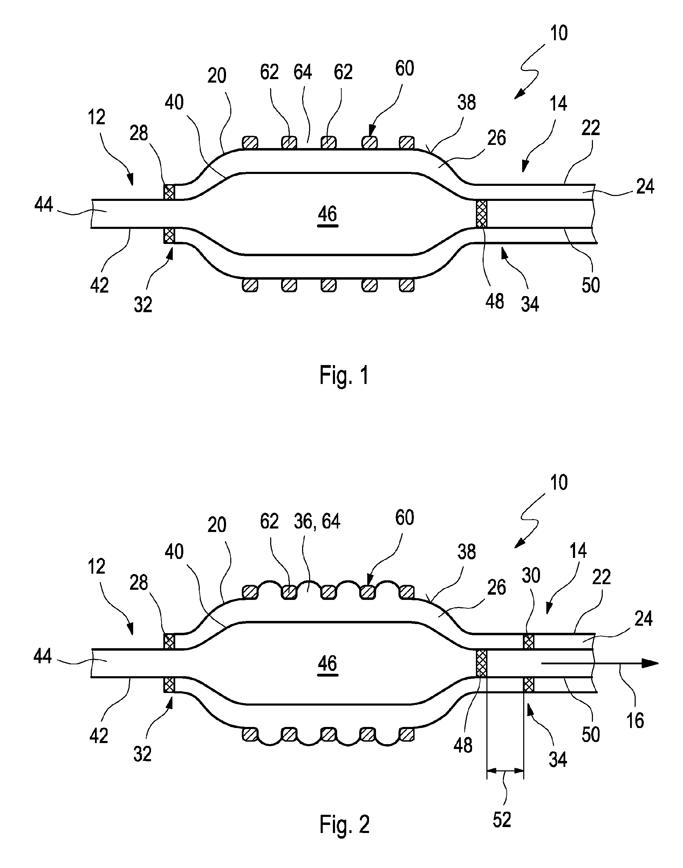

Fig. 1 und Fig. 2 zeigen im Schnitt eine erste bevorzugte Ausgestaltung eines beispielhaft als Doppelmembranballon ausgebildeten Mehrfachmembranballons 10, der mit einem Stützorgan 60 (Stent) umgeben ist, in zwei verschiedenen Verfahrensschritten eines bevorzugten Herstellverfahrens. Fig. 1 zeigt den Doppelmembranballon (im weiteren Kontext mit 10 bezeichnet) in einem ersten Verfahrensschritt während der Herstellung mit losem Stützorgan 60, Fig. 2 zeigt den Doppelmembranballon 10 mit befestigtem Stützorgan 60. Die Membranhüllen 20, 40 können aus üblichen, geeigneten Materialien gebildet sein wie z.B. Gummi oder Kunststoff, wie etwa Polyamid, Polyester, wie Polyethylenterephthalat (PET).

Der Doppelmembranballon 10 umfasst eine äußere, gemäß dem dargestellten Verfahrensschritt einseitig geschlossene äußere Membranhülle 20, in der eine innere, einseitig geschlossene Membranhülle 40 angeordnet ist, welche einen befüll- und entleerbaren Ausdehnungsraum 46 zwischen einem proximalen Ende 12 und einem distalen Ende 14 des Doppelmembranballons 10 aufweist. Die äußere Membranhülle 20 ist mit einem Zwischenraum 26 zu der inneren Membranhülle 40 beabstandet. Die äußere Membranhülle 20 kann außenseitig mit einer Schutzschicht 38 beschichtet sein, welche z.B. die Gleitfähigkeit verbessern und/oder auch die Membranhülle 20 vor Beschädigungen schützen kann.

Die innere Membranhülle 40 ist am distalen Ende 14 geschlossen, die äußere Membranhülle 20 ist an ihrem dem proximalen Ende 12 zugewandten Ende 32 geschlossen und liegt mit einem Verschluss 28 dicht an einem Tubus 42 an. Der Verschluss 28 kann z.B. mit Verschweißen hergestellt sein. Das steife Stützorgan 60 umschließt die doppelte Membranhülle 20, 40.

Am proximalen Ende 12 ist an der inneren Membranhülle 40 der Tubus 42 angeordnet. Durch einen Hohlraum 44 des Tubus 42 kann die innere Membranhülle 40 bzw. deren Ausdehnungsraum 46 am Einsatzort mit einem geeigneten Medium aufgeblasen und der Doppelmembranballon 10 folglich aufgeweitet werden. Die innere Membranhülle 40 ist am distalen Ende 14 mit einem Verschluss 48 abgedichtet. Der Verschluss 48 kann z.B. durch Verschweißen gebildet sein. An den Verschluss 48 grenzt ein Tubus 50 an, in dem z.B. ein Führungsdraht oder andere Komponenten angeordnet sein können (nicht dargestellt).

Die äußere Membranhülle 20 ist in dem in Fig. 1 dargestellten Verfahrensschritt an ihrem dem distalen Ende 14 zugewandten Ende 34 noch offen und geht in einen Tubus 24 über, der einen Hohlraum 24 aufweist und der den Tubus 50 umschließt.

Vorteilhaft kann die äußere Membranhülle 20 eine größere Berstgrenze aufweisen als die eine oder die mehreren inneren Membranhülle 40, so dass im ungünstigen Fall eines Hüllbruchs der innern Membranhülle 40 die Membranhülle 20 stabil bleiben kann.

Die äußere Membranhülle 20 kann so gefertigt sein, dass sie beim Aufweiten der inneren Membranhülle 40 denselben Dehnungszustand wie diese aufweist. Damit ist der Mehrfachmembranballon 10 in sich sehr stabil, und die günstigen festigkeitserhöhenden Eigenschaften der Doppel- bzw. Mehrfachmembranhüllen können ausgenutzt werden, da die inneren und äußeren Membranhüllen 40, 20 nunmehr gleichmäßig gedehnt werden können.

Die äußere Membranhülle 20 kann zweckmäßigerweise ein größeres Volumen aufweisen als die eine innere Membranhülle 40 im regulären aufgeweiteten Zustand, so dass bei einem Platzen der Inhalt der inneren Membranhülle 40 sicher von der äußeren Membranhülle 20 aufgenommen werden kann.

Entsprechend einem bevorzugten Verfahrensschritt des erfindungsgemäßen Verfahrens, der in Fig. 2 skizziert ist, wird die äußere Membranhülle 20 aufgeblasen bzw. aufgeweitet, bis die äußere Membranhülle 20 das diese umgebende Stützorgan 60 wenigstens teilweise durchsetzt, bevorzugt bis die äußere Membranhülle 20 formschlüssig mit dem Stützorgan 60 verbunden ist. Die äußere Membranhülle 20 ragt dabei in der Art von Noppen oder Falten 36 in Zwischenräume 64 von Streben 62 des Stützorgans 60.

Nachdem das Stützorgan 60 in die äußere Membran 20 eingebettet ist, kann das Stützorgan 60 auf einen gewünschten geringern Durchmesser komprimiert werden, z.B. durch Crimpen, den es z.B. beim Einführen zum Einsatzort einnehmen soll. Der Mehrfachmembranballon 10 stellt für den Crimpvorgang in Form des Tubus 24 ein distales Ballonlumen für das Aufweiten der äußeren Membranhülle 20 zur Verfügung. Das distale Ballonlumen wird nach dem erfolgten Einbetten des Stützorgans 60 nahe dem Ballon verschlossen und abgetrennt.

Die äußere Membranhülle 20 ragt in der Art von Falten oder Noppen 36 in Zwischenräume 64 von Streben 62 des Stützorgans 60. Dadurch sitzt das Stützorgan 60 besonders stabil auf dem Mehrfachmembranballon 10 und weist eine hohen Widerstand gegen ein Abstreifen auf, so dass die Abzugskraft, um das Stützorgan 60 vom Mehrfachmembranballon 10 abzuziehen, stark erhöht ist. Wird der Mehrfachmembranballon 10 am Einsatzort auf seinen Einsatzdurchmesser aufgeweitet, indem ein geeignetes Medium in den Ausdehnungsraum 46 der inneren Membranhülle 40 geleitet wird, weitet sich auch die äußere Membranhülle 20 und das Stützorgan 60. Ist der Solldurchmesser des Stützorgans 60 erreicht, kann der Ausdehnungsraum 46 wieder entleert werden, so dass das Stützorgan 60 dann leicht vom Mehrfachmembranballon 10 abgestreift werden kann.

Nach dem Einbetten und Komprimieren des Stützorgans 60 kann die äußere Membranhülle 20 an ihrem offenen Ende 34 verschlossen und abgetrennt werden. Vorteilhaft ist, wenn die Membranhülle 20 bzw. der Zwischenraum 26 vorher evakuiert wird. Dadurch werden die Streben 62 des Stützorgans 60 besonders gut zwischen den Noppen 36 der äußeren Membranhülle 20 fixiert. Dadurch, dass das komprimierte Stützorgan 60 in die äußere Membranhülle 20 eingebettet ist, bilden diese für die innere Membranhülle 40 eine relativ dicke Schutzummantelung, so dass bei der Handhabung des Mehrfachmembranballons 10 die innere Membranhülle 40 besonders gut geschützt ist.

Beim Verschließen der äußeren Membranhülle 20 an ihrem distalen Ende 34 wird vorzugsweise der distale Verschluss 30 der äußeren Membranhülle 20 in axialer Richtung 16 hinter einem distalen Verschluss 48 der inneren Membranhülle 40 angeordnet. Dies führt dazu, dass der Mehrfachmembranballon 10 im Halsbereich sehr flexibel ist. Würden die Verschlüsse 48 der inneren Membranhülle 40 und 30 der äußeren Membranhülle 20 aufeinander liegen, wäre dieser Bereich des Mehrfachmembranballons 10 sehr steif.

Günstig ist, wenn der distale Verschluss 30 der äußeren Membranhülle 20 in axialer Richtung 16 mit einem Abstand 52 hinter dem distalen Verschluss 48 der inneren Membranhülle 40 liegt, der wenigstens der einfachen axialen Erstreckung, vorzugsweise wenigstens der doppelten axialen Erstreckung des Verschlusses 30 entspricht.

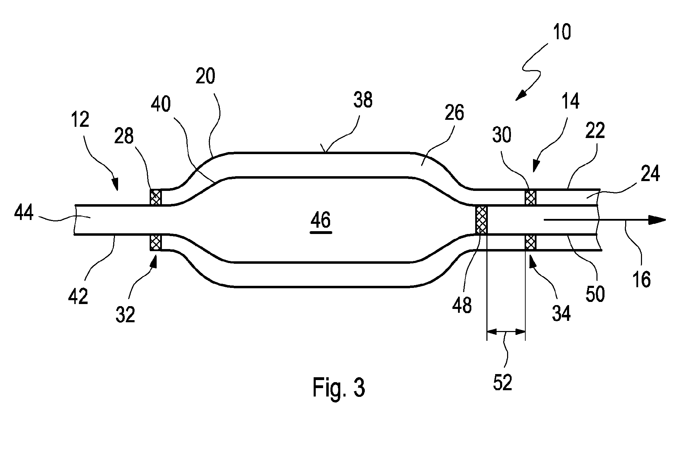

Ein weiterer Aspekt der Erfindung ist in Fig. 3 dargestellt. Der Aufbau des Mehrfachmembranballons 10 entspricht demjenigen, der in den Fig. 1 und 2 dargestellt ist. Im Gegensatz dazu ist jedoch kein Stützorgan vorgesehen, das den Mehrfachmembranballon 10 umgibt. Zu nicht erläuterten Details, die in beiden Darstellungen des Mehrfachmembranballons 10 vorhanden sind, wird zur Vermeidung unnötiger Wiederholungen daher auf die vorherigen Figurengeschreibungen verwiesen.

Die äußere Membranhülle 20 ist an ihrem distalen Ende 32 und ihrem proximalen Ende 34 verschlossen, wobei der distale Verschluss 30 der äußeren Membranhülle 20 in axialer Richtung 16 hinter einem distalen Verschluss 48 der innere Membranhülle 40 liegt.

Der distale Verschluss 30 der äußeren Membranhülle 20 in axialer Richtung 16 liegt günstigerweise mit einem Abstand 52 hinter dem distalen Verschluss 48 der inneren Membranhülle 40, der vorzugsweise wenigstens der einfachen axialen Erstreckung des Verschlusses 30 entspricht. Durch den axialen Versatz ist der Halsbereich des Mehrfachmembranballons 10 mit den Verschlüssen 48 und 30 sehr flexibel und kann sich leicht Krümmungen anpassen. Ein Einführen zum Einsatzort kann dadurch vereinfacht werden.

Auch in diesem Fall kann ein Zwischenraum 26 zwischen der äußeren Membranhülle 20 und der inneren Membranhülle 40 evakuiert sein, um der Anordnung mehr Stabilität zu verleihen und die innere Membranhülle 40 mechanisch zu schützen.

## Patentansprüche

1. Mehrfachmembranballon für einen Katheter, mit einer wenigstens doppelten Membranhülle (20, 40), umfassend wenigstens einen befüll- und entleerbaren Ausdehnungsraum (46) zwischen einem proximalen Ende (12) und einem distalen Ende (14), wobei die äußere Membranhülle (20) an ihrem distalen Ende (32) und ihrem proximalen Ende (34) verschlossen ist und eine oder mehrere innere Membranhüllen (40) umschließt, **dadurch gekennzeichnet, dass** die äußere Membranhülle (20) in der Art von Falten oder Noppen (36) in Zwischenräume (64) der Streben eines die äußere Membranhülle (20) umgebenden Stützorgans (60) ragt.

2. Mehrfachmembranballon nach Anspruch 1, **dadurch gekennzeichnet, dass** die äußere Membranhülle (20) formschlüssig mit dem Stützorgan (60) verbunden ist.

3. Mehrfachmembranballon nach einem der Ansprüche 1 oder 2, **dadurch gekennzeichnet, dass** der distale Verschluss (32) der äußeren Membranhülle (20) in axialer Richtung (16) vorzugsweise mit einem Abstand (52) überlappungsfrei hinter einem distalen Verschluss (48) der einen oder mehreren inneren Membranhüllen (40) liegt.

4. Mehrfachmembranballon nach einem der vorhergehenden Ansprüche, **dadurch gekennzeichnet, dass** ein Zwischenraum (26) zwischen der äußeren Membranhülle (20) und der nächstfolgenden inneren Membranhülle (40) einen Druck unterhalb von Atmosphärendruck aufweist.

5. Mehrfachmembranballon nach einem der vorhergehenden Ansprüche, **dadurch gekennzeichnet, dass** die äußere Membranhülle (20) eine größere Berstgrenze aufweist als die eine oder die mehreren inneren Membranhülle (40) und so gefertigt ist, dass sie beim Aufweiten der einen oder der mehreren inneren Membranhüllen (40) denselben Dehnungszustand aufweist.

6. Mehrfachmembranballon nach einem der vorhergehenden Ansprüche, **dadurch gekennzeichnet, dass** die äußere Membranhülle (20) an ihrer Außenseite und/oder Innenseite wenigstens bereichsweise eine Schutzschicht (38) aufweist.

7. Mehrfachmembranballon nach einem der vorhergehenden Ansprüche, **dadurch gekennzeichnet, dass** die äußere Membranhülle (20) ein größeres Volumen aufweist als die eine oder die mehreren inneren Membranhüllen (40) im regulären aufgeweiteten Zustand.

8. Mehrfachmembranballon nach einem der vorhergehenden Ansprüche, der für einen Crimpvorgang des Stützorgans (60) ein distales Ballonlumen für ein Aufweiten der äußeren Membranhülle (20) zur Verfügung stellt.

9. Verfahren zur Herstellung eines Mehrfachmembranballons (10) nach einem der Ansprüche 1 bis 8 , mit einer wenigstens doppelten Membranhülle (20, 40), **dadurch gekennzeichnet, dass** eine äußere Membranhülle (20) der wenigstens doppelten Membranhülle (20, 40) aufgeweitet wird, bis die äußere Membranhülle (20) ein die äußere Membranhülle (20) umgebendes Stützorgan (60) wenigstens teilweise durchsetzt, dass die äußere Membranhülle (20) mit dem Stützorgan (60) formschlüssig verbunden wird und dass das Stützorgan (60) auf einen gewünschten geringeren Durchmesser komprimiert wird.

10. Verfahren nach Anspruch 9, **dadurch gekennzeichnet, dass** die äußere Membranhülle (20) an ihrem offenen Ende (34) verschlossen wird oder dass die Membranhülle (20) evakuiert und an ihrem offenen Ende (34) verschlossen wird.

11. Verfahren nach Anspruch 9 oder 10, **dadurch gekennzeichnet, dass** der Mehrfachmembranballon (10) für einen Crimpvorgang des Stützorgans (60) ein distales Ballonlumen für ein Aufweiten der äußeren Membranhülle (20) zur Verfügung stellt, wobei das distale Ballonlumen nach dem Einbetten des Stützorgans (60) verschlossen und gekürzt wird.

12. Verfahren nach einem der Ansprüche 9 bis 11, **dadurch gekennzeichnet, dass** die Membranhüllen (20, 40) so hergestellt werden, dass sie im aufgeweiteten Zustand einen gleichartigen Dehnungszustand einnehmen.

## Claims

1. A multi-membrane balloon for a catheter, having an at least double membrane sleeve (20, 40), comprising at least one expansion space (46), which can be filled and emptied, between a proximal end (12) and a distal end (14), wherein the outer membrane sleeve (20) is closed at its distal end (32) and its proximal end (34) and encloses one or more inner membrane sleeves (40), **characterised in that** the outer membrane sleeve (20) protrudes in the manner of folds or nubs (36) into gaps (64) between the struts of a supporting element (60) surrounding the outer membrane sleeve (20).

2. The multi-membrane balloon according to Claim 1, **characterised in that** the outer membrane sleeve (20) is connected in a form-fitting manner to the supporting element (60).

3. The multi-membrane balloon according to one of Claims 1 or 2, **characterised in that** the distal closure (32) of the outer membrane sleeve (20) lies in the axial direction (16) preferably at a distance (52) without overlap behind a distal closure (48) of the one or more inner membrane sleeves (40).

4. The multi-membrane balloon according to one of the preceding claims, **characterised in that** a gap (26) between the outer membrane sleeve (20) and the next inner membrane sleeve (40) has a pressure below atmospheric pressure.

5. The multi-membrane balloon according to one of the preceding claims, **characterised in that** the outer membrane sleeve (20) has a higher bursting limit than the one or more inner membrane sleeves (40) and is manufactured such that it has the same elongation state when the one or more inner membrane sleeves (40) is/are dilated.

6. The multi-membrane balloon according to one of the preceding claims, **characterised in that** the outer membrane sleeve (20) on its outer side and/or inner side has a protective layer (38) at least in regions.

7. The multi-membrane balloon according to one of the preceding claims, **characterised in that** the outer membrane sleeve (20) has a greater volume than the one or more inner membrane sleeves (40) in a regular dilated state.

8. The multi-membrane balloon according to one of the preceding claims, which has a distal balloon lumen for dilation of the outer membrane sleeve for a crimping operation of the supporting element (60).

9. A method for producing a multi-membrane balloon (10) according to one of Claims 1 to 8, having an at least double membrane sleeve (20, 40), **characterised in that** an outer membrane sleeve (20) of the at least double membrane sleeve (20, 40) is dilated until the outer membrane sleeve (20) at least partially penetrates a supporting element (60) surrounding the outer membrane sleeve (20), **in that** the outer membrane sleeve (20) is connected in a form-fitting manner to the supporting element (60), and **in that** the supporting element (60) is compressed to a desired smaller diameter.

10. The method according to Claim 9, **characterised in that** the outer membrane sleeve (20) is closed at its open end (34), or **in that** the membrane sleeve (20) is evacuated and is closed at its open end (34).

11. The method according to Claim 9 or 10, **characterised in that** the multi-membrane balloon (10) provides a distal balloon lumen for dilation of the outer membrane sleeve (20) for a crimping operation of the supporting element (20), wherein the distal balloon lumen is closed and shortened once the supporting element (60) has been embedded.

12. The method according to one of Claims 9 to 11, **characterised in that** the membrane sleeves (20, 40) are produced such that in the dilated state they have a uniform state of elongation.

## Revendications

1. Ballonnet à membranes multiples pour un cathéter, avec au moins une double enveloppe de membrane (20, 40), comprenant au moins une chambre de dilatation (46) pouvant être remplie et vidée entre une extrémité proximale (12) et une extrémité distale (14), dans lequel l'enveloppe de membrane (20) externe est fermée à son extrémité distale (32) et à son extrémité proximale (34) et entoure une ou plusieurs enveloppes de membrane (40) interne, **caractérisé en ce que** l'enveloppe de membrane (20) externe dépasse comme des plis ou des noeuds (36) dans des espaces intermédiaires (64) des entretoises d'un organe support (60) entourant l'enveloppe de membrane (20) externe.

2. Ballonnet à membranes multiples selon la revendication 1, **caractérisé en ce que** l'enveloppe de membrane (20) externe est reliée avec l'organe support (60) par complémentarité des formes.

3. Ballonnet à membranes multiples selon l'une des revendications 1 ou 2, **caractérisé en ce que** la fermeture distale (32) de l'enveloppe de membrane (20) externe se situe dans la direction axiale (16) de préférence sans recouvrement avec un espace (52) derrière une fermeture distale (48) de la ou des plusieurs enveloppes de membrane (40) internes.

4. Ballonnet à membranes multiples selon l'une des revendications précédentes, **caractérisé en ce qu'**une chambre intermédiaire (26) entre l'enveloppe de membrane (20) externe et l'enveloppe de membrane (40) interne suivante présente une pression en dessous d'une pression atmosphérique.

5. Ballonnet à membranes multiples selon l'une des revendications précédentes, **caractérisé en ce que** l'enveloppe de membrane (20) externe présente une plus grande limite d'éclatement que celle de la ou des plusieurs enveloppes de membrane (40) internes et est ainsi fabriquée qu'elle présente le même état d'allongement que la ou les plusieurs enveloppes de membrane (40) internes lors de l'élargissement.

6. Ballonnet à membranes multiples selon l'une des revendications précédentes, **caractérisé en ce que** l'enveloppe de membrane (20) externe présente au moins localement une couche de protection (38) sur sa face externe et/ou sa face interne.

7. Ballonnet à membranes multiples selon l'une des revendications précédentes, **caractérisé en ce que** l'enveloppe de membrane (20) externe présente un volume plus grand que celui de la ou des plusieurs enveloppes de membrane (40) internes à l'état élargi régulier.

8. Ballonnet à membranes multiples selon l'une des revendications précédentes, qui met à disposition un lumen de ballonnet distal pour un élargissement de l'enveloppe de membrane (20) externe pour une opération de sertissage de l'organe support (60).

9. Procédé de fabrication d'un ballonnet à membranes multiples (10) selon l'une des revendications 1 à 8, avec au moins une double enveloppe de membrane (20, 40), **caractérisé en ce qu'**une enveloppe de membrane (20) externe parmi l'au moins double enveloppe de membrane (20, 40) est élargie jusqu'à ce que l'enveloppe de membrane (20) externe traverse au moins partiellement un organe support (60) entourant l'enveloppe de membrane (20) externe, que l'enveloppe de membrane (20) externe se trouve reliée par complémentarité des formes avec l'organe support (60) et que l'organe support (60) est comprimé jusqu'à un diamètre plus petit souhaité.

10. Procédé selon la revendication 9, **caractérisé en ce que** l'enveloppe de membrane (20) externe est fermée à son extrémité ouverte (34) ou que l'enveloppe de membrane (20) est évacuée et fermée à son extrémité ouverte (34).

11. Procédé selon les revendications 9 ou 10, **caractérisé en ce que** le ballonnet à membranes multiples (10) met à disposition un lumen de ballonnet distal pour un élargissement de l'enveloppe de membrane (20) externe pour un processus de sertissage de l'organe support (60), où le lumen de ballonnet distal est fermé et raccourci après l'intégration de l'organe support (60).

12. Procédé selon l'une des revendications 9 à 11, **caractérisé en ce que** les enveloppes de membrane (20, 40) sont fabriquées de telle manière qu'elles adoptent un état de dilatation semblable à l'état élargi.
